# EUROPÄISCHE PATENTSCHRIFT

(11) **EP 0 587 101 B1**
(45) Veröffentlichungstag und Bekanntmachung des Hinweises auf die Patenterteilung: **12.03.1997**
(21) Anmeldenummer: 93114297.0
(22) Anmeldetag: 07.09.1993
(51) Int. Cl.: A61M 1/34, A61M 1/36, A61K 31/725

(54) **Vorrichtung zur Entfernung von Aluminiumionen aus Blut und Lösung zur Verwendung in der Vorrichtung**
Apparatus for the removal of aluminium ions from blood and solution used in this apparatus
Dispositif pour l'élimination des ions aluminium du sang et solution utilisée dans ce dipositif

(30) Priorität: 11.09.1992 DE 4230513
(43) Veröffentlichungstag der Anmeldung: 16.03.1994
(73) Patentinhaber: Fresenius AG, D-61350 Bad Homburg (DE)
(72) Erfinder: Mathieu, Bernd, Dr., D-66583 Spiesen-Elversberg (DE)
(74) Vertreter: Fuchs, Luderschmidt & Partner Patentanwälte

(56) Entgegenhaltungen:
- WO-A-91/06326
- DE-A- 4 004 978
- ASAIO TRANSACTIONS, Bd.34, Nr.3, Juli 1988, HAGERSTOWN,MD US Seiten 524 - 527, XP000045322 'CITRATE FOR REGIONAL ANTICOAGULATION'

## Beschreibung

Die Erfindung betrifft eine Vorrichtung zur Entfernung von Aluminiumionen aus Blut gemäß dem Oberbegriff des Anspruchs 1. Desweiteren betrifft die Erfindung eine Lösung zur Verwendung in der erfindungsgemäßen Vorrichtung, wie sie in den Ansprüchen 15 und 17 beschrieben ist.

Seit mehr als 20 Jahren ist es bekannt, daß sich Aluminium in den Knochen von Dialysepatienten ablagert und daher die Notwendigkeit besteht, Aluminium vom Körper dieser Patienten fernzuhalten bzw. es wieder zu entfernen.

Bekanntlich erfolgt die Ausscheidung von Aluminium bei Gesunden nahezu ausschließlich über die Niere. Insofern sind Patienten ohne eigene Nierenfunktion hierzu nicht in der Lage und sammeln das Aluminium in ihrem Körper an, falls die Aufnahme von Aluminium die Ausscheidung durch die im allgemeinen dreimal pro Woche durchgeführte Dialysebehandlung übersteigt.

Hauptquellen für die Aluminiumaufnahme sind Phosphatbinder auf Aluminium-Hydroxyd-Basis, Trinkwasser, Speisen, die in Aluminiumgefäßen zubereitet oder aufbewahrt worden sind sowie diverse Pharmazeutika.

Die Ausscheidung von Aluminium über die Membran eines Dialysators während der Dialyse ist unter normalen Umständen relativ gering. So wird selbst bei modernen offenporigen High-Flux-Dialysatoren nur eine Reduktion des Aluminiumwerts um maximal 40 % des Ausgangswerts bei der Passage durch den Dialysator erreicht. Infolgedessen lagert sich das restliche Aluminium in den Knochen ab, was zu Knochenzysten und Knochenerweichung führt. Nachdem nahezu jeder Patient nach 5-jähriger Dialyseabhängigkeit mehr oder weniger starke Aluminiumablagerungen in Knochen und Gewebe aufweist, führt dies zu einer erheblichen negativen Beeinträchtigung des Allgemeinzustands der Patienten.

Aus der DE-OS 40 04 978 ist es bekannt, zur präventiven Therapie von Morbus Alzheimer Aluminium-Ionen aus dem Organismus zu eliminieren. Dies kann durch perorale Verabreichung von synthetischen Komplexbildnern erreicht werden, insbesondere von Schutzgruppen aufweisenden Hydroxyaromaten. Die Affinität der Komplexbildner zu Aluminium-Ionen ist dabei höher als diejenige von Citrat oder von ATP.

Aufgrund ihrer oralen Verabreichung und der dadurch bedingten systemischen Wirkung kann es jedoch zu erheblichen Nebenwirkungen kommen.

Außerdem wurden bereits Versuche unternommen (Hümpfner, A., "Aluminiumintoxikation bei Niereninsuffizienz", Zuckschwerdt Verlag, München 1989), auch die Reinigungsleistungen von Dialysatoren mit Hilfe von Chelatbildnern, beispielsweise Desferroxamin (DFO), zu erhöhen. Solche Chelatbildner wurden den Patienten entweder oral verabreicht und wirkten somit ebenfalls systemisch oder sie wurden über einen Perfusor appliziert oder entweder i.m., langsam i.v., subkutan oder auch intraperitoneal appliziert.

Obwohl mit der Verabreichung von DFO gute Anfangserfolge erzielt worden sind, konnte eine chronische Behandlung nicht durchgeführt werden, da sich DFO als zu toxisch erwies. Es war also unmöglich, das aufgenommene Aluminium praktisch zeitgleich, d.h. innerhalb der nächsten Dialysebehandlung, wieder zu entfernen. Insofern ging man dazu über, die DFO-Verabreichung nur noch in bestimmten Zeiträumen (Kuren) durchzuführen, bei denen das Aluminium aus den Knochen mobilisiert und anschließend mit Hilfe der Dialyse entfernt wurde. Nachteilig an solchen Behandlungen ist jedoch die Tatsache, daß erst eine relativ hohe Aluminiumkonzentration im Knochen erreicht werden muß und daß in der Zeit zwischen der Gabe des DFO und der Entfernung des Al-DFO-Komplexes durch Dialysebehandlung (üblicherweise am folgenden Tag) erhebliche Konzentrationen von Aluminium (vorwiegend als DFO-Komplex) im Blut auftreten, was zu massiven Nebenwirkungen führt.

Schon 1961 wurde von Morita et al. (Am. J. Med. Sci., 242, Seiten 32 ff.) über Versuche zur Verwendung von Trinatriumcitrat zur regionalen Antikoagulation bei der Dialysebehandlung berichtet.

Unter Vermeidung von Heparin als Antikoagulationsmittels wurde das Trinatriumcitrat dem Blutkreislauf durch Infusion unmittelbar vor Durchströmung des Dialysators zugeführt.

Aufgrund des vollständigen Verzichts auf Heparin sind bei der vorgeschlagenen Methode zur Vermeidung einer ggfls. auftretenden Aktivierung des Blutgerinnungssystems an der Fremdoberfläche des Leistungssystems des extrakorporalen Blutkreislaufs entsprechend hohe Dosen an Trinatriumcitrat zur sicheren Antikoagulation nötig.

Bei den heute üblichen hohen Flußraten von 200-300 ml/min führt dies zu einem Eintrag von großen Mengen Citrat in den Körper des Patienten. Der Eintrag kann zwar durch intravenöse Kalziumgabe ausgeglichen werden, was allerdings eine verfahrenstechnische Erschwernis darstellt.

Darüber hinaus besteht bei zu großem Trinatrium-Citrat-Eintrag ins Blut die Möglichkeit eines unerwünschten Anstiegs des Blutnatriumwertes, mit der möglichen Folge eines entstehenden Bluthochdrucks.

Aufgrund dessen schien die Verwendung von Citrat bei der Dialysebehandlung nicht ratsam.

In der gattungsbildenden WO 91/06326 ist er Ersatz von Heparin durch Citrat erneut aufgegriffen worden, wobei ebenfalls stromab vom Dialysator dem Blutkreislauf Citrat als Antikoagulans zugesetzt wird. Zur Vermeidung von Alkalosen, infolge des Citronensäureabbaus zu CO₂ und H₂O im Stoffwechsel, wurden bei dem in der WO beschriebenen Verfahren aufwendige Zusatzmaßnahmen ergriffen. U.a. wird ein Dialysat mit Natrium- und Calciumkonzentrationen unterhalb der Normalwerte liegend verwendet und eine Gesamtalkalikonzentration, die hinreichend zum Schutz vor Alkalosen ist.

Angesichts des dargelegten Standes der Technik liegt der Erfindung die Aufgabe zugrunde, eine Vorrichtung der eingangs erwähnten Art so fortzubilden, daß sie die Reinigung des Bluts von Aluminiumionen während der Dialysebehandlung erlaubt, ohne daß ernsthafte Nebenwirkungen beim Patienten zu befürchten sind. Desweiteren hat die Erfindung die Aufgabe Lösungen bereitzustellen, die in der Vorrichtung zur Entfernung der Aluminiumionen eingesetzt werden können.

Die Lösung der Aufgabe erfolgt durch eine Vorrichtung mit den Merkmalen des kennzeichnenden Teils des Anspruchs 1 und durch Lösungen zur Verwendung in der Vorrichtung mit den Merkmalen des kennzeichnenden Teils der Ansprüche 15 oder 17.

Vorteilhafte Ausgestaltungen der erfindungsgemäßen Vorrichtung werden in den jeweiligen abhängigen Ansprüchen unter Schutz gestellt.

Mit der erfindungsgemäßen Vorrichtung gelingt es, Aluminiumionen aus Vollblut zu entfernen, wobei dem Blutkreislauf stromauf vom semipermeablem Filter sowohl mindestens ein Antikoagulationsmittel als auch mindestens eine Substanz zu Ausbildung einer komplexen Bindung mit den zu entfernenden Aluminiumionen zugefügt wird.

Dabei ist es im Rahmen der Erfindung prinzipiell auch möglich, daß der oder die Komplexbildner auch auf weitere im Blut vorhandene Metallionen komplexierend wirken. Es wird jedoch angestrebt, eine möglichst große Reaktivität des Komplexierungsmittels gegenüber Aluminiumionen und eine besonders gute Stabilität des gebildeten Komplexes mit einer hohen Selektivität der Komplexbildung insbesondere in Bezug auf Aluminiumionen zu vereinen.

Nichtsdestotrotz erlaubt es das Prinzip der Erfindung aber auch, neben einer effektiven Entfernung von Aluminiumionen, bei geeigneter Wahl des Komplexierungsmittels und der Reaktionsbedingungen, andere im Blut vorhandene Metallionen wirkungsvoll zu entfernen.

Es ist jedoch bevorzugt, das System bezüglich seiner Fähigkeit der selektiven Aluminiumionenelimination optimiert zu verwenden.

Wichtig ist, daß durch die in der erfindungsgemäßen Vorrichtung zu verwendenden Substanzen eine genügend hohe Metallionenentfernung, insbesondere für Aluminiumionen, mit einer ausreichenden Gerinnungshemmung auch stromab vom semipermeablen Filter gewährleistet wird.

Aufgrund der Wechselwirkung zwischen Metallionen und Komplexbildner oder Komplexbildnern entstehen energetisch stabile Verbindungen, die wegen ihrer molekularen Dimensionen und des Konzentrationsgefälles zwischen Blutkreislaufseite und Dialyseseite des Filters durch das Filter diffundieren und mittels der Dialysierflüssigkeit abgeführt werden können.

Die Vorrichtung zur Entfernung von Aluminiumionen aus Blut kennzeichnet sich vorteilhaft dadurch, daß die Aluminiumionen komplexierende Lösung exakt mit einer Dosiervorrichtung, beispielsweise einer Pumpe, dosiert werden kann. Hierfür kann beispielsweise eine Rollerpumpe verwendet werden, welche zweckmäßig zwischen Quelle, also Reservoir des Komplexbildners, und Anschluß des Reservoirs an die Blutzuführleitung des Blutkreislaufs angeordnet ist.

Die Zufuhr von vorbestimmten Mengen an Aluminiumionen komplexierender Lösung ist aus zwei Gründen besonders vorteilhaft.

Zum einen muß abgestimmt auf die Aktivität bzw. Konzentration der zu entfernenden Aluminiumionen im Blut, welche in der Regel ebenfalls nicht in freier Form, sondern in komplex gebundener Form (beispielsweise an Einweißkörper wie Albumin o.ä.) vorliegen, und abgestimmt auf die Stabilität der Bindung des gegebenenfalls im Blut bereits vorliegenden Aluminiumionen- Eiweißkörper-Komplexes, sowie abgestimmt auf die Blutflußrate im Blutkreislauf, eine zur Entfernung der Aluminiumionen ausreichende Menge an geeignetem Komplexbildner zudosiert werden.

Zum anderen darf die zudosierte Komplexbildnermenge nicht beliebig groß sein, da im allgemeinen in der erfindungsgemäßen Vorrichtung zwar im wesentlichen nicht toxische Komplexbildner eingesetzt werden, aber selbst erwiesenermaßen atoxische Substanzen bei einem zu hohen Eintrag ins Blut des Patienten nachteilige Nebenwirkungen entfalten können. Der Eintrag des Komplexbildners ins Blut kann allerdings nicht nur durch eine nicht erfindungsgemäße Überdosierung, sondern auch durch eine mangelnde Diffusionsfähigkeit eines Aluminiumionenkomplexes verursacht werden. Beispielsweise ist bei Verwendung eines Membranfilters die Möglichkeit gegeben, daß trotz ausreichender Bildungsgeschwindigkeit und Stabilität des Aluminiumionenkomplexes dieser eine mangelnde Membrangängigkeit aufweist. Daher ist die Art des Komplexbildners in solch einem Fall auch auf das verwendete (Membran-) Filter abzustimmen.

In einer bevorzugten Ausführungsform der erfindungsgemäßen Vorrichtung ist die Verbindung von Blutzuführleitung mit der Quelle für die Aluminiumionen komplexierende Lösung mit Distanz zum Anschluß der Blutzuführleitung des Blutkreislaufs an das semipermeable Filter angeordnet. Der Abstand oder diese Distanz dient zur Ausbildung einer Durchmischungsstrecke für das Blut und die zudosierte Aluminiumionen komplexierende Lösung. Die Verbindung von Blutzuführleitung und Leitung für die Aluminiumionen komplexierende Lösung ist dabei soweit vor dem semipermeablen Filter anzuordnen, daß abgestimmt auf die Flußrate des Blutes in der Blutzuführleitung und die Zufuhrgeschwindigkeit und das Volumen der Aluminiumionen komplexierenden Lösung eine innige Durchmischung zwischen Blut und Lösung erreicht werden kann. Eine ausreichende Durchmischung wiederum ist je nach Geschwindigkeit der Komplexbildung, Stabilität des Komplexes und möglichen Konkurrenzreaktionen Voraussetzung für die Komplexbildung zwischen Aluminiumionen und Komplexbildner. Insbesondere bei Ausbildung von Strömungsprofilen in der Blutzuführleitung kommt einer geeigneten Länge der Durchmischungsstrecke eine wesentliche Bedeutung zu.

Man kann zwar die Strömung in der Blutzuführleitung turbulent führen, es ist jedoch bevorzugt, das semipermeable Filter bzw. einen eingangsseitigen Anschluß des semipermeablen Filters mit einem speziell geformten Mischanschluß bzw. einer Mischvorrichtung oder Mischkammer zu versehen, um Blut sowie die mit der Quelle, welche Aluminiumionen komplexierende Lösung enthält, verbundene Leitung an den Mischanschluß oder die Mischkammer anzuschließen.

Im letzteren Fall kann auf eine längere Mischstrecke in der Blutzuführleitung verzichtet werden, wodurch die Vorrichtung als solche kompakter ausgeführt sein kann. Letztlich muß jedoch immer eine ausreichende Durchmischung vor Eintritt des Blutkreislaufs in das semipermeable Filter gewährleistet sein.

Ein Beispiel für eine zur Mischung geeignete Vorrichtung ist u.a. eine sogenannten Proportional-Mischeinheit, wie sie dem Fachmann aus der Verwendung in Dialysiersystemen geläufig ist. Weiterhin geeignet sind Tropfkammern oder auch die bekannten Misch-Bilanzierungssysteme. Die Dosage in das Blutschlauchsystem kann u.a. mit einer steuerbaren Rollenpumpe oder einer steuerbaren Spritzenpumpe vollzogen werden.

Als semipermeables Filter können in der erfindungsgemäßen Vorrichtung alle Filter eingesetzt werden, die für die jeweils zu entfernenden Aluminiumionenkomplexe halb durchlässig sind, d.h. die für die zu entfernenden Komplexe nur in einer Richtung durchlässig sind. Bevorzugt sind dies Membranfiltermedien, deren Verwendung dem Fachmann beispielsweise in Dialysatoren gemäß dem Stand der Technik bekannt ist.

Wie bereits weiter oben ausgeführt, besteht ein wichtiger Aspekt der Erfindung darin, daß das Aluminiumionen komplexierende Mittel zusätzlich zu einem Antikoagulationsmittel eingesetzt wird. Ebenso wie eine genaue Dosage des Aluminiumionen komplexierenden Mittels ist in einer weiteren Ausführungsform auch die genaue Dosierung des Antikoagulans bevorzugt. Zweckmäßig geschieht dies dadurch, daß zwischen Blutzuführleitung und der damit verbundenen Quelle des Antikoagulationsmittels eine Pumpe geschaltet ist, mit der dem Blutkreislauf aus der Quelle vorbestimmte Mengen Antikoagulationsmittel zuführbar sind.

Die Menge des zuzuführenden Antikoagulationsmittels richtet sich nach Menge und Beschaffenheit des Blutes. Da in einigen Fällen das Aluminiumionen komplexierende Mittel auch antikoagulatorisch wirken kann, beispielsweise über Komplexierung von für die Gerinnungshemmung essentiellen Metallionen, kann die Menge an Antikoagulationsmittel, die verwendet werden muß, auf das Einzelproblem abgestimmt auch geringer sein, als bei alleiniger Verwendung eines einzigen Antikoagulationsmittels.

Wenn das Antikoagulationsmittel dem Blutkreislauf weiter stromauf vom semipermeablen Filter zugeführt wird als das unter Umständen ebenfalls gerinnungshemmend wirkende Aluminiumionen komplexierende Mittel, dann muß das Antikoagulationsmittel zumindest die Gerinnung des Blutes durch Kontakt mit den Fremdflächen des Leitungssystems des Blutkreislaufs bis zum Zutritt des ebenfalls antikoagulatorisch wirkenden Aluminiumionen komplexierenden Gemisches verhindern.

Der Mischpunkt des Antikoagulationsmittels mit dem Blut kann allerdings auch mit dem Mischpunkt für die Aluminiumionen komplexierende Lösung zusammenfallen. Bevorzugt ist aber die Zugabe des Antikoagulans stromauf vom Aluminiumionenkomplexbildner.

Als Antikoagulationsmittel kommen alle Stoffe in Frage, die üblicherweise zur Gerinnungshemmung von Blut verwendet werden. Hierzu zählen u.a. Heparin, Hirudin und/oder Prostacyclin etc.

Ganz besonders bevorzugt ist erfindungsgemäß jedoch die Verwendung von Heparin als Antikoagulans, das in an sich bekannter Weise die Blutgerinnung im Leitungssystem des Blutkreislaufs verhindert. Da Heparin eine polydisperse makromolekulare Substanz ist, wird hierbei in der Regel eine höhermolekulare, im wesentlichen nicht dialysierbare Heparin-Fraktion mit einem Molekulargewicht von MW > 20.000 verwendet.

Als Aluminiumionen komplexierende Lösungen kommen alle üblicherweise zu diesem Zwecke einsetzbaren Lösungen anorganischer oder organischer Komplexbildner in Frage. Dabei spielt die Art des gebildeten Komplexes eine untergeordnete Rolle. Es können Chelate, Cluster, Brücken-, Sandwich-, Kronen-, oder Podandenverbindungen u.ä. sein. Wesentlich ist allerdings, daß der gebildete Komplex dialysierbar ist. Zu den Komplexbildnern gehören beispielsweise EDTA, Oxalat, DFO, im wesentlichen membrangängiges Heparin und/oder Citrat.

Zu berücksichtigen sind in der Regel die Toxizität bzw. die möglichen Nebenwirkungen des Komplexbildners auf einen Patienten. Spielt dieser Umstand jedoch keine ausschlaggebende Rolle, so sind angepaßt an das jeweils zu lösende Problem alle genannten Substanzen gleichberechtigt. Spielt jedoch die Verträglichkeit für einen Organismus die ausschlaggebende Rolle, so ist die Verwendung von Citrat, insbesondere Trinatriumcitrat, bei weitem bevorzugt.

Desweiteren bevorzugt ist der Einsatz von membrangängigen Heparin mit einem Molekulargewicht MW<5000.

In alternativer Ausführungsform ist Gegenstand der Erfindung auch eine Vorrichtung zur Entfernung von Aluminiumionen aus Blut mit
- einem semipermeablen Filter, das zwei Seiten aufweist,
- einem Blutzuführ- und Blutrückführleitung aufweisenden Blutkreislauf, der auf eine Seite des Filters gelegt ist,
- einer Dialysierflüssigkeitsleitung, die auf die andere Seite des Filters gelegt ist,
- einer Dialysierflüssigkeitsquelle, die mit der Dialysierflüssigkeitsleitung verbunden ist, und
- einer mit der Blutzuführleitung verbundenen Quelle eines Antikoagulationsmittels, das im wesentlichen nicht dialysierbar ist, wobei die Quelle zusätzlich auch ein Aluminiumionen komplexierendes Mittel aufweist.

Zur Vereinfachung der Apparatur ist es nun durchaus zweckmäßig, eine Ausführungsform der Vorrichtung vorzusehen, in der die Quellen des Antikoagulationsmittels und des Komplexbildners in einer gemeinsamen Quelle zusammenfallen. Damit ist im Rahmen der Erfindung gemeint, daß Komplexbildner und Antikoagulans aus einer einzigen Quelle, bzw. Reservoir, in Form einer gemeinsamen Lösung zugegeben werden können. Dies ist allerdings nur dann sinnvoll, solange die beiden Substanzen sich in einer Lösung nicht gegenseitig nachteilig beeinflussen, und solange die Konzentrationen von Komplexbildner und Antikoagulans während einer Anwendung in der erfindungsgemäßen Vorrichtung im wesentlichen ein konstantes Verhältnis bilden können.

Beim Vorliegen einer gemeinsamen Quelle ist es bevorzugt, daß die gemeinsame Quelle dem Blutkreislauf vorbestimmt zuführbare Mengen von Citrat und Heparin als im wesentlichen nicht dialysierbaren Antikoagulationsmittel aufweist.

Eine weitere bevorzugte Kombination bei Vorliegen einer einzigen Quelle besteht im gleichzeitigen Einsatz von im wesentlichen nicht dialysierbarem Heparin und von membrangängigem Heparin.

Wie bereits weiter oben angedeutet, können antikoagulatorische Substanzen auch komplexierend wirken und umgekehrt.

Beim Sonderfall des Heparins nun, bei dem ein polydisperser Stoff die Möglichkeit offeriert, in Abhängigkeit von der verwendeten Molekulargewichtsfraktion die Membrangängigkeit stark zu beeinflussen, gewährleistet die vorgenannte bevorzugte Kombination sowohl die Aufrechterhaltung einer ausreichenden Gerinnungshemmung stromauf und stromab vom semipermeablen Filter als auch den angestrebten Austrag von Aluminiumionen.

Neben der Möglichkeit, nur eine gemeinsame Quelle zu verwenden und auch einen polymeren Stoff, allerdings in verschiedenen Fraktionen bezüglich seines Molekulargewichts, bietet die vorliegende Erfindung des weiteren vorteilhaft die Möglichkeit, auch nur eine Fraktion eines polydispersen Stoffes zu verwenden. In einer Ausführungsform der Erfindung ist es deshalb bevorzugt, lediglich Heparin in nur einer Quelle einzusetzen. Durch einen genügend hohen Eintrag eines besonders kurzkettigen Heparins (MW < 5000), wird eine genügend große Metallionenmenge aus dem Blutkreislauf über die Dialysierflüssigkeit entfernt. Der Anteil des Heparins, der nicht entfernt wird, gewährleistet stromab des Filters die Antikoagulation.

Die Vorrichtung gemäß der Erfindung ist prinzipiell dazu geeignet, je nach verwendetem Komplexierungsmittel eine Vielzahl von im Blut vorhandenen Metallionen aus dem Blut zu entfernen. Zu diesen Metallionen gehören u.a. Magnesiumionen, Kalziumionen, Aluminiumionen, Eisenionen und/oder Kupferionen. Diese können entweder von einem Komplexbildner oder auch gleichzeitig von einem Gemisch von Komplexbildnern aus dem Blut entfernt werden.

Es ist jedoch aufgrund der in der Einleitung gemachten Ausführungen ganz besonders bevorzugt, mit der erfindungsgemäßen Vorrichtung Aluminiumionen aus dem Blut zu entfernen.

Geschieht dies während einer Dialyse unter gleichzeitiger Verwendung von Citrat als Komplexbildner und Heparin als Antikoagulationsmittel, so wird im Dialysator der Aluminium-Citratkomplex, sowie ggfls. überschüssiges Citrat zusammen mit Urämietoxinen über die semipermeable Membran an die Dialysierflüssigkeit abgegeben und dadurch aus dem Blut entfernt.

In diesem Fall kann es bevorzugt sein, daß die Dialysierflüssigkeitsquelle eine Dialysierflüssigkeit mit Konzentrationen an Metallionen aufweist, die ggfls. unerwünscht auf demselben Weg dem Blut entzogen wurden.

Falls der Komplexbildner also nicht spezifisch genug wirkt, kann man über diesen Weg Metallionen resubstituieren.

Da die Entfernung von Kupfer, Kalzium und Magnesium bei einer üblichen Dialysierbehandlung nicht angestrebt wird, ist es vorteilhaft, daß die Dialysierflüssigkeitsquelle Konzentrationen an Kupfer-, Kalzium- und/oder Magnesium-Ionen, ganz besonders bevorzugt jedoch an Kalzium-Ionen aufweist, um die jeweiligen freien Ionen aus dem Dialysat zu ergänzen, die ggfls. vom Komplexbildner unerwünscht gebunden wurden und demzufolge durch das Filter entfernt wurden. Es versteht sich, daß zum Zwecke dieser Substitution die Ionen in ausreichender Konzentration in der Dialysierflüssigkeitslösung vorhanden sein sollen. In Ausnahmefällen kann allerdings auch ein oraler Ausgleich, beispielsweise bei der Substitution von Eisenionen, zweckmäßig sein.

Die Diffusion der komplexierten Metallionen von der Blut- und Dialysierseite kann allein aufgrund von Konzentrationsunterschieden vonstatten gehen. Sie wird jedoch besonders wirkungsvoll dadurch unterstützt, daß bei einer erfindungsgemäßen Vorrichtung in der Dialysierflüssigkeitsleitung eine Ultrafiltrationspumpe angeordnet ist. Diese ist so steuerbar, daß dem Blutkreislauf nur so viel Ultrafiltrat entzogen wird, wie ihm Flüssigkeit während der Dialysebehandlung mit der Vorrichtung zugeführt worden ist, einschließlich der zusätzlichen Mengen an Lösung Aluminiumionen komplexierenden Mittels. Dabei ist die Ultrafiltrationspumpe steuerbar, ganz gleich, ob aus einer oder aus zwei Quellen Metallionen komplexierende Lösung und Antikoagulationslösung zugegeben wird.

Die erfindungsgemäße Vorrichtung gestattet demnach in ihren einzelnen Ausführungformen die Durchführung eines kombinierten Antikoagulations-Komplexierungsverfahrens, das sich wesentlich von der eingangs beschriebenen regionalen Citrat-Antikoagulation unterscheidet. Die regionale Citrat-Antikoagulation muß mit kalzium- und magnesiumfreien Dialysaten arbeiten, um eine Koagulation im Leitungs- bzw. Schlauchsystem nach dem Dialysator zu verhindern. Bei einem Verfahren mit der erfindungsgemäßen Vorrichtung wird die Konzentration des Antikoagulationsmittels (beispielsweise des Heparins) kaum verringert und die zusätzliche systemische Gabe von Kalzium und Magnesium ist nicht notwendig.

Die Erfindung betrifft auch Lösungen zur Verwendung in den einzelnen Ausführungsformen der erfindungsgemäßen Vorrichtung. Insbesondere stellt die Erfindung Aluminiumionen komplexierende und antikoagulatorische Lösungen zur Verfügung, die sich durch einen Gehalt an Citrationen und einen Gehalt an im wesentlichen nicht membrangängigem Heparin kennzeichnen. Des weiteren ist es vorteilhaft, erfindungsgemäß eine Lösung zu verwenden, die gleichzeitig membrangängiges und im wesentlichen nicht membrangängiges Heparin aufweist. Auch werden erfindungsgemäß Lösungen verwendet, die eine erforderliche Menge an membrangängigem Heparin aufweisen.

Die Gehalte an Citrat und/oder Heparin der erfindungsgemäß zu verwendenden Lösungen können über einen gemeinsamen Bereich variieren.

So sind citrathaltige Lösungen mit ca. 40-50 g/l Natriumcitrat und 14-18 g/l Citronensäure-Monohydrat bevorzugt. Besonders bevorzugt sind Lösungen mit Gehalten von 44 g/l Natriumcitrat und 16 g/l Zitronensäure-Monohydrat.

Die letztgenannten Gehalte entsprechen einer Citratkonzentration von ca. 0,25 M. Bei der Mischung der Lösung mit Blut beträgt das Mischungsverhältnis erfindungsgemäß wenigstens 1:100 (=2,5 mM Citrat) und höchstens 1:5 (=50 mM Citratlösung). Ist das Mischverhältnis kleiner als 1:100, so ist die Komplexbildung mit Aluminiumionen nicht mehr gewährleistet. Ist das Mischungsverhältnis größer als 1:5, so führt die hohe Citratkonzentration im Blut zu unerwünschten Nebenwirkungen, die eingangs bereits erwähnt wurden. Bevorzugt ist ein Mischungsverhältnis von ca. 1:7 (=35 mM).

Die Lösungsgehalte an hochmolekularem Heparin betragen bei der erfindungsgemäßen Verwendung zwischen 3 und 200 mg/l. Bevorzugt zwischen 30 und 50 mg/l und besonders bevorzugt 40 mg/l.

Hochmolekulares Heparin (MG > 5000, vorzugsweise MG = 20 000) wird in einer Boluseinheit von 5000 E an den Patienten gegeben (= 38,5 mg/l). Ebenso wird das Blutschlauchsystem mit 5000 E pro Liter Kochsalzlösung vorgefüllt. Pro Stunde werden 1000 E an den Patienten gegeben, um die Anfangskonzentration aufrechtzuerhalten. Liegt die Konzentration an hochmolekularem Heparin unter 3 mg/l in der erfindungsgemäß einzusetzenden Lösung, so kann eine Koagulation nicht mehr zuverlässig vermieden werden; höhere Konzentrationen als 200 mg/l sind ggfls. toxisch.

Niedermolekulares Heparin kann in der erfindungsgemäß zu verwendenden Lösung in einem Konzentrationsbereich von ca. 3 mg/l bis ca. 600 mg/l enthalten sein. Gehalte von 30 bis 50 mg/l sind bevorzugt. Besonders vorteilhaft ist ein Gehalt von ca. 40 mg/l. Niedermolekulares Heparin (MG < 5000, z.B. MG = 4500) kann allerdings auch in relativ höheren Konzentrationen (bis 600 mg/l) verabreicht werden als hochmolekulares Heparin.

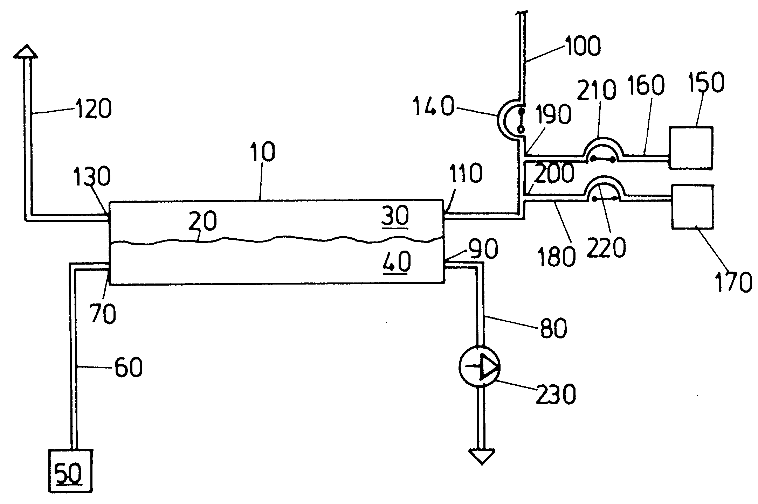

Im folgenden werden der Aufbau und die Wirkungsweise einer erfindungsgmäßen Vorrichtung anhand der Figur erläutert.

Die Figur zeigt eine schematische Darstellung einer Ausführungsform eines Dialysators mit den angeschlossenen Leitungen.

Mit 10 ist der Dialysator bezeichnet. Im Dialysator 10 ist eine semipermeable Membran 20 angeordnet. Diese trennt den Dialysator 10 in eine Blutseite 30 und eine Dialysatseite 40. Blutseite 30 und Dialysatseite 40 sind gegeneinander leckdicht und nur über die semipermeable Membran 20 verbunden.

Des weiteren ist in der Figur eine Dialysierflüssigkeitsquelle 50 erkennbar. Diese ist über eine Leitung 60 mit einem Einlaß 70 der Dialysatseite 40 des Dialysators 10 verbunden. Die Leitung 80 ist mit einem Auslaß 90 der Dialysatseite 40 des Dialysators 10 in Verbindung.

Eine Blutzuführleitung 100 ist an einen Einlaß 110 der Blutseite 30 des Dialysators 10 angeschlossen. Eine Blutabführleitung 120 ist an einen Auslaß 130 der Blutseite 30 des Dialysators 10 angeschlossen. In der Blutzuführleitung 100 ist stromauf vom Dialysator 10 eine Pumpe 140 angeordnet.

Neben der Dialysierflüssigkeitsquelle 50 sind noch zwei weitere Quellen in der Figur zu erkennen. Eine Quelle 150 eines Antikoagulationsmittel ist über eine Leitung 160 mit der Blutzuführleitung 100 und eine weitere Quelle 170 eines Metallionen komplexierenden Mittels ist über eine Leitung 180 ebenfalls mit der Blutzuführleitung 100, aber stromab der Quelle 150, verbunden. Der Verbindungspunkt der Quelle 150 mit der Blutzuführleitung 100 ist mit 190, der Verbindungspunkt der Quelle 170 mit der Blutzuführleitung 100 ist mit 200 bezeichnet. Sowohl zwischen Quelle 150 und Verbindungspunkt 190 als auch zwischen Quelle 170 und Verbindungspunkt 200 ist jeweils eine Pumpe 210 bzw. 220 zwischengeschaltet. Stromab des Dialysators 10 ist in der Leitung 80 eine Ultrafiltrationspumpe 230 angeordnet.

Die Vorrichtung wird nun folgendermaßen betrieben: Im extrakorporalen Blutkreislauf bestehend aus Leitung 100, Pumpe 140, Blutseite 30 des Dialysators 10 und Leitung 120 wird Vollblut zirkuliert. Dies kann beispielsweise einem Dialysepatienten entnommen werden. Im Gegenstrom dazu wird die Dialyseflüssigkeit aus der Quelle 50 über Leitungen 60 und 80 sowie die Dialysatseite 40 des Dialysators 10 mittels der Ultrafiltrationspumpe 230 an der Membran 20 entlang geführt. An der semipermeablen Membran 20 kommt es aufgrund unterschiedlicher Konzentrationen der Vollblut und der Dialysatlösung zum Stoffaustausch durch Diffusion. Der Blutzuführleitung 100 wird stromab der Pumpe 140 aus der Quelle 150 über Leitung 160 und Pumpe 210 am Mischpunkt 190 Heparin als Antikoagulanz zudosiert. Stromab vom Verbindungspunkt 190 aber mit ausreichender Durchmischungsstrecke 200-110 wird aus der Quelle 170 über Leitung 180 und Pumpe 220 eine vorbestimmte Menge Trinatriumcitrat als Komplexbildner zugesetzt. Heparin verhindert dabei die Gerinnung im extrakorporalen Kreislauf, während Citrat im Blut vorhandene Aluminiumionen komplexiert und dialysierbar macht.

Die Effektivität der Vorrichtung wird im folgenden anhand eines Beispiels verdeutlicht:

### Beispiel:

6 Liter frisches Rinderblut werden mit etwa 6000 E (= 1000 E/l = 7,7 mg) hochmolekularem Heparin (Heparin-Natrium MW 20000, FRESENIUS AG) und im Verhältnis 1:7 mit einer handelsüblichen ACD-Lösung (gerinnungshemmende Lösung als Konservierungszusatz zu Frischblut; enthält Acidum citricum purum 2,5 %, Dextrose 2,34 % und Natrium citricum 2,16 %) versetzt und mit physiologischer Kochsalzlösung auf einen Hämatokrit von 30 eingestellt. Das Blut wird auf 37°C thermostatiniert und dabei gerührt. 20 mg Aluminium (als Aluminiumsulfat) werden in 1 Liter physiologischer Kochsalzlösung aufgelöst und von dieser Lösung ca. 10 ml/L (entsprechend 0,2 mg/l Blut) zugesetzt. Das Blut wird weitere 30 min bei 37°C gerührt. Ein Kreislauf wird aufgebaut aus einem Reservoir für 3 L Blut, einem Reservoir für 3 L physiologischer Kochsalzlösung, einem Dialysator (im vorliegenden Faslle ein HEMOFLOW F60). Das Schlauchsystem besitzt in Strömungsrichtung des Blutes folgende Teile:
- ein Schlauchstück vom Reservoir der phys. Kochsalzlösung zu einem Pumpsegment,
- ein Pumpsegment, das in einer Rollerpumpe eingelegt ist,
- ein Schlauchstück von diesem Pumpsegment to dem Dialysator,
- ein Dialysator, dessen Dialyseflüssigkeits-Bereich mit 500 ml physiologischer Kochsalzlösung von 37°C durchströmt wird,
- ein Schlauchstück vom Dialysator zurück zum Reservoir.

Die Rate der Rollerpumpe wird auf 50 ml/min eingestellt und das System blasenfrei gefüllt.

Anschließend wird die Pumpe angehalten und der Eingang des Schlauchsystems in das Reservoir des heparinisierten Rinderblutes, der Ausgang des Schlauchsystems in ein leeres Gefäß eingebracht. Die Pumpe wird gestartet und das Blut mit 50 ml/min durch das System gepumpt. Nach 10 Minuten werden simultan Proben genommen am Eingang und am Ausgang des Schlauchsystems (Probe Cit50ein und Cit50aus). Die Flußrate wird danach auf 200 ml erhöht und nach weiteren 10 Minuten erneut Proben genommen, am Eingang und am Ausgang gleichzeitig (Proben Cit200ein und Cit200aus). Eine Probe der Dialysierflüssigkeit wird vor dem Eingang in den Dialysator genommen und mit CitDial bezeichnet.

### Vergleichsbeispiel

6 Liter frisches Rinderblut werden mit 125.000 Einheiten Heparin (Heparin-Natrium MW 20.000, FRESENIUS AG) in 250 ml phys. Kochsalzlösung versetzt und mit weiterer physiologischer Kochsalzlösung auf einen Hämatokrit von 30 eingestellt. Das Blut wird auf 37°C thermostatisiert und dabei gerührt. 20 mg Aluminium (als Aluminiumsulfat) werden in 1 Liter physiologischer Kochsalzlösung aufgelöst und von dieser Lösung ca. 10 ml/L (entsprechend 0,2 mg/l Blut) zugesetzt. Blut wird weitere 30 min bei 37°C gerührt.

Der weitere Verlauf des Versuchs entspricht exakt dem des Beispiels. Die Proben werden mit Hep50ein und Hep50aus, bzw. Hep200ein und Hep200aus bezeichnet, die Kontrollmessung der verwendeten Dialysierflüssigkeit mit HepDial.

### Ergebnis:

| | | | | | |
|---|---|---|---|---|---|
| ppm/l Al | HepDial < 2 | Hep50ein 146 | Hep50aus 80 | Hep200ein 140 | Hep200aus 83 |
| ppm/l Al | CitDial < 2 | Cit50ein 131 | Cit50aus 40 | Cit200aus 145 | Cit200aus 52 |
| Antikogul. Blutfluß | Heparin 50 ml/min | 200 ml/min | Citrat 50 ml/min | 200 ml/min | |
| Reinigungsleistung | 41 % | 40,7 % | 69,4 % | 64,1 % | |

Ein Vergleich der Reinigungsleistung resultiert in einer deutlichen Überlegenheit der kombinierten Verwendung von Citrationen und Heparin.

### Bezugszeichenliste

- 10: Dialysator
- 20: semipermeable Membran
- 30: Blutseite
- 40: Dialysatseite
- 50: Dialyseflüssigkeitsquelle
- 60: Leitung
- 70: Einlaß
- 80: Leitung
- 90: Auslaß
- 100: Blutzuführleitung
- 110: Einlaß
- 120: Blutabführleitung
- 130: Auslaß
- 140: Pumpe
- 150: Quelle eines Antikoagulationsmittels
- 160: Leitung
- 170: Quelle eines metallkomplexierenden Mittels
- 180: Leitung
- 190: Verbindungspunkt
- 200: Verbindungspunkt
- 210: Pumpe
- 220: Pumpe
- 230: Ultrafiltrationspumpe

## Patentansprüche

1. Vorrichtung zur Entfernung von Aluminiumionen aus Blut mit
- einem semipermeablen Filter (20), das zwei Seiten (30, 40) aufweist,
- einem Blutzuführ- (100) und Blutrückführleitung (120) aufweisenden Blutkreislauf (100, 30, 120), der auf eine Seite (30) des Filters (20) gelegt ist,
- einer Dialysierflüssigkeitsleitung (60, 80), die auf die andere Seite (40) des Filters (20) gelegt ist,
- einer Dialysierflüssigkeitsquelle (50), die mit der Dialysierflüssigkeitsleitung (60) verbunden ist und
- einer mit der Blutzuführleitung (100) verbundenen Quelle (150) eines Antikoagulationsmittels, das im wesentlichen nicht dialysierbar ist,
dadurch gekennzeichnet, daß stromauf des Filters (20)
- eine weitere Quelle (170) eines Aluminiumionen komplexierenden Mittels über eine Zuführleitung (180) mit der Blutzuführleitung (100) verbunden ist.

2. Vorrichtung nach Anspruch 1, dadurch gekennzeichnet, daß in die Zuführleitung (180) eine Pumpe (220) eingeschaltet ist, mittels der dem Blutkreislauf (100, 30, 120) vorbestimmte Mengen Aluminiumionen komplexierenden Mittels als Lösung zuführbar ist.

3. Vorrichtung nach Anspruch 1 oder 2, dadurch gekennzeichnet, daß die Verbindung (200) von Blutzuführleitung (100) und Zuführleitung (180) der weiteren Quelle (170) zur Ausbildung einer Durchmischungsstrecke (200-110) für Blut und Aluminiumionen komplexierende Lösung mit Distanz zu einem Anschluß (110) der Blutzuführleitung (100) an der Blutseite (30) des semipermeablen Filters (20) angeordnet ist.

4. Vorrichtung nach Anspruch 1 oder 2, dadurch gekennzeichnet, daß die Blutseite (30) des semipermeablen Filters (20) einen zur Mischung von Blut und Aluminiumionen komplexierender Lösung geformten Anschluß aufweist.

5. Vorrichtung nach einem der vorhergehenden Ansprüche, dadurch gekennzeichnet, daß das semipermeable Filter (20) ein Membranfilter in einem Dialysator (10) ist.

6. Vorrichtung nach einem der vorhergehenden Ansprüche, dadurch gekennzeichnet, daß Blutzuführleitung (100) und die Quelle eines Antikoagulationsmittels (150) über eine Leitung (160) verbunden sind, in die eine Pumpe (210) eingeschaltet ist, mittels der dem Blutkreislauf (100,30,120) aus der Quelle (150) vorbestimmte Mengen Antikoagulationsmittel zuführbar sind.

7. Vorrichtung nach einem der vorhergehenden Ansprüche, dadurch gekennzeichnet, daß die Quelle (150) eines Antikoagulationsmittels Heparin, Hirudin und/oder Prostacyclin, bevorzugt Heparin, aufweist.

8. Vorrichtung nach einem der vorhergehenden Ansprüche, dadurch gekennzeichnet, daß die weitere Quelle (170) einer Metallionen komplexierenden Lösung EDTA, Oxalat, DFO, im wesentlichen membrangängiges Heparin und/oder Citrat, bevorzugt Citrat, aufweist.

9. Vorrichtung zur Entfernung von Aluminiumionen aus Blut mit
- einem semipermeablen Filter (20), das zwei Seiten (30, 40) aufweist,
- einem Blutzuführ- (100) und Blutrückführleitung (120) aufweisenden Blutkreislauf (100, 30, 120), der auf eine Seite (30) des Filters (20) gelegt ist,
- einer Dialysierflüssigkeitsleitung (60, 80), die auf die andere Seite (40) des Filters (20) gelegt ist,
- einer Dialysierflüssigkeitsquelle (50), die mit der Dialysierflüssigkeitsleitung (60) verbunden ist, und
- einer mit der Blutzuführleitung (100) verbundenen Quelle (150) eines Antikoagulationsmittels, das im wesentlichen nicht dialysierbar ist,
dadurch gekennzeichnet, daß
- die Quelle (150) zusätzlich auch ein Aluminiumionen komplexierendes Mittel aufweist.

10. Vorrichtung nach Anspruch 9, dadurch gekennzeichnet, daß die Quelle (150) dem Blutkreislauf (100,30,120) vorbestimmt zuführbare Mengen von Citrat und von Heparin aufweist.

11. Vorrichtung nach Anspruch 9, dadurch gekennzeichnet, daß die Quelle (150) dem Blutkreislauf (100,30,120) vorbestimmt zuführbare Mengen von im wesentlichen nicht dialysierbarem Heparin und von membrangängigem Heparin aufweist.

12. Vorrichtung nach einem der vorhergehenden Ansprüche, dadurch gekennzeichnet, daß die Dialysierflüssigkeitsquelle (50) eine Dialysierflüssigkeit mit Konzentrationen an Metallionen aufweist, die zur Substitution von unerwünscht aus dem Blutkreislauf (100,30,120) entfernten komplexierten Metallionen geeignet sind.

13. Vorrichtung nach Anspruch 12, dadurch gekennzeichnet, daß die Dialysierflüssigkeitsquelle (50) Konzentrationen an Kupfer-, Kalzium- und/oder Magnesiumionen, bevorzugt Kalziumionen, aufweist.

14. Vorrichtung nach einem der vorhergehenden Ansprüche, dadurch gekennzeichet, daß stromab des Dialysators (10) in der Dialysierflüssigkeitsleitung (80) eine Ultrafiltrationspumpe (230) angeordnet ist, die so steuerbar ist, daß dem Blutkreislauf (100,30,120) zusätzlich zu vorbestimmten Mengen Ultrafiltrat Ultrafiltratmengen entziehbar sind, die den Mengen Aluminiumionen komplexierender Lösung entsprechen, die dem Blutkreislauf aus einer der Quellen (150,170) zuführbar ist.

15. Aluminiumionen komplexierende und antikoagulatorische Lösung zur Verwendung in einer Vorrichtung gemäß einem der Ansprüche 9 bis 11 oder 12 bis 14 in Verbindung mit einem der Ansprüche 9 bis 11, gekennzeichnet durch einen Gehalt an Citrationen und einen Gehalt an im wesentlichen nicht membrangängigem Heparin.

16. Lösung nach Anspruch 15, gekennzeichnet durch einen Natriumcitratgehalt von 40-50 g/l, bevorzugt 44 g/l, einen Zitronensäure-Monohydrat-Gehalt von 14-18 g/l, bevorzugt 16 g/l, und einen Gehalt an hochmolekularem Heparin (MG > 5000) von 30-50 mg/l, bevorzugt 40 mg/l.

17. Aluminiumionen komplexierende und antikoagulatorische Lösung zur Verwendung in einer Vorrichtung gemäß einem der Ansprüche 9 bis 11 oder 12 bis 14 in Verbindung mit einem der Ansprüche 9 bis 11, gekennzeichnet durch Gehalte an membrangängigem Heparin und im wesentlichen nicht membrangängigem Heparin.

18. Verwendung der Lösung gemäß einem der Ansprüche 15 bis 17 in einer Vorrichtung gemäß einem der Ansprüche 9 bis 11 oder 12 bis 14 in Verbindung mit einem der Ansprüche 9 bis 11.

## Claims

1. Apparatus for the removal of aluminium ions from blood with
- a semi-permeable filter (20) having two sides (30, 40),
- a blood supply conduit (100) and a blood return back conduit (120) having a blood circuit (100, 30, 120) which is placed on one side (30) of the filter (20),
- a dialysis liquid conduit (60, 80) which is placed on the other side of the filter (20),
- a dialysis liquid source (50) which is connected to the dialysis liquid conduit (60) and
- a source (150) of an anticoagulation medium which essentially is not dialysable connected to the blood supply conduit (100), characterised in that downstream of the filter (20),
- a further source (170) of an aluminium ion complexing medium is connected via a supply conduit (180) to the blood supply conduit (100).

2. Apparatus according to claim 1, characterised in that in the supply conduit (180) a pump (220) is inserted by means of which the predetermined quantities of aluminium ion complexing medium is supported as solution to the blood circuit (100, 30, 120).

3. Apparatus according to claim 1 or 2, characterised in that the connection (200) of blood supply conduit (100) and supply circuit conduit (180) of the further source (170) for the forming of a mixing line (200-110) for blood and aluminium ion complexing solution with distance to a connection (110) of the blood supply conduit (100) is disposed on the blood side (30) of the semi-permeable filter (20).

4. Apparatus according to claim 1 or 2, characterised in that the blood side (30) of the semi-permeable filter (20) has a connection formed for the mixing of blood and aluminium ion complexing solution.

5. Apparatus according to one of the preceding claims, characterised in that the semi-permeable filter (20) is a membrane filter in a dialyser (10).

6. Apparatus according to one of the preceding claims, characterised in that the blood supply conduit (100) and the source of an anticoagulation medium (150) are connected via a conduit (160) in which a pump (210) is inserted by means of which the predetermined quantities of anticoagulation medium are supplied to the blood circuit (100, 30, 120) from the source (150).

7. Apparatus according to one of the preceding claims, characterised in that the source (150) of an anticoagulation medium has heparin, hirudin and/or prostacyclin, preferably heparin.

8. Apparatus according to one of the preceding claims, characterised in that the further source (170) of a metal ion complexing solution EDTA. Oxalate, DFO has essentially membrane usable heparin and/or citrate preferably citrate.

9. Apparatus for the removal of aluminium ions from blood, with
- a semi-permeable filter (20) having two sides (30, 40),
- a blood circuit (100, 30, 120) having a blood conduit (100) and a blood return conduit (120) which blood circuit is placed on one side (30) of the filter (20),
- a dialysis liquid conduit (60, 80) which is placed on the other side (40) of the filter (20),
- a dialysis liquid source (60) which is connected to the dialysis liquid conduit (60) and
- a source (150) of an anticoagulation medium which is essentially non-dialysisable, which source is connected to the blood supply conduit (100), characterised in that
- the source (150) has additionally also an aluminium complexing medium.

10. Apparatus according to claim 9, characterised in that the source (150) has predetermined quantities of citrate and of heparin supplied to the blood circuit (100, 30, 120).

11. Apparatus according to claim 9, characterised in that the source (150) has predetermined quantities of essentially non-dialysisable heparin and of membrane usable heparin conveyable to the blood circuit (100, 30, 120).

12. Apparatus according to one of the preceding claims, characterised in that the dialysis liquid source (50) has a dialysis liquid with concentrations of metal ions which are suitable for the substitution of undesired complexed metal ions removed from the blood circuit (100, 30, 120).

13. Apparatus according to claim 12, characterised in that the dialysis liquid source (50) has concentrations of copper; calcium and/or magnesium ions, preferably calcium ions.

14. Apparatus according to one of the preceding claims, characterised in that downstream of the dialyser (10) there is disposed in the dialysis liquid conduit (80) an ultra-filtration pump (230) which is so controllable that additionally to predetermined quantities of ultrafiltrate, ultrafiltrate quantities are extractable from the blood circuit, which correspond to the quantities of aluminium ion complexing solution which is supplied to the blood circuit from one of the sources (150, 170).

15. An aluminium ion complexing and anticoagulatoric solutions for use in an apparatus according to one of the claims 9 to 11, or 12 to 14 in conjunction with one of the claims 9 to 11, characterised by a content of citrate ions and a content of essentially non-membrane usable heparin.

16. A solution according to claim 15, characterised by a sodium nitrate content of 40-50 g/l, preferably 44 g/l, a citric acid-monohydrate content of 14-18 g/l preferably 16 g/l and a content of high molecular heparin (MG > 5000) of 30-50 mg/l, preferably 40 mg/l.

17. An aluminium ion complexing and anticoagulatoric solution for use in an apparatus according to one of the claims 9 to 11 or 12 to 14 in conjunction with one of the claims 9 to 11, characterised by contents of membrane usable heparin and essentially non-membrane usable heparin.

18. Use of the solution according to one of the claims 15 to 17 in an apparatus according to one of the claims 9 to 11 or 12 to 14 in conjunction with one of the claims 9 to 11.

## Revendications

1. Dispositif pour éliminer les ions aluminium du sang à l'aide
- d'un filtre semi-perméable (20), qui présente deux côtés (30, 40),
- d'une circulation sanguine (100, 30, 120) présentant une conduite d'amenée de sang (100) et une conduite de recyclage de sang (120), laquelle circulation sanguine est disposée d'un côté (30) du filtre (20),
- d'une conduite de liquide de dialyse (60, 80), qui est disposée de l'autre côté (40) du filtre (20),
- d'une source de liquide de dialyse (50) qui est reliée à la conduite de liquide de dialyse (60), et
- d'une source (150) d'un agent anticoagulant relié à la conduite d'amenée de sang (100), lequel est essentiellement non dialysable, caractérisé en ce que, en amont du filtre (20),
- une autre source (170) d'un agent complexant les ions aluminium est reliée par l'intermédiaire d'une amenée (180) à la conduite d'amenée de sang (100).

2. Dispositif selon la revendication 1, caractérisé en ce qu'à la conduite d'amenée (180) est connectée une pompe (220), à l'aide de laquelle on peut amener à la circulation sanguine (100, 30, 120), des quantités prédéterminées d'agent complexant les ions aluminium sous forme de solution.

3. Dispositif selon la revendication 1 ou 2, caractérisé en ce que la liaison (200) de la conduite d'amenée de sang (100) et de la conduite d'amenée (180) de l'autre source (170) est disposée pour former une section de brassage (200-110) du sang et de la solution complexant les ions aluminium avec une distance à un raccord (110) de la conduite d'amenée de sang (100) du côté sang (30) du filtre semi-perméable (20).

4. Dispositif selon la revendication 1 ou 2, caractérisé en ce que le côté sang (30) du filtre semi-perméable (20) présente un raccord formé pour mélanger le sang et la solution complexant les ions aluminium.

5. Dispositif selon l'une des revendications précédentes, caractérisé en ce que le filtre semi-perméable (20) est un filtre membranaire dans un dialyseur (10).

6. Dispositif selon l'une des revendications précédentes, caractérisé en ce que la conduite d'amenée de sang (100) et la source d'un agent anticoagulant (150) sont reliées par l'intermédiaire d'une conduite (160), dans laquelle est installée une pompe (210) à l'aide de laquelle on peut amener des quantités prédéterminées d'agents anticoagulants à partir de la source (150) au circuit sanguin (100, 30, 120).

7. Dispositif selon l'une des revendications précédentes, caractérisé en ce que la source (150) présente un agent anticoagulant, l'héparine, l'hirudine et/ou la prostacycline, de préférence l'héparine.

8. Dispositif selon l'une des revendications précédentes, caractérisé en ce que l'autre source (170) présente une solution complexant les ions métalliques EDTA, l'oxalate, DFO, essentiellement l'héparine dialysable et/ou le citrate, de préférence le citrate.

9. Dispositif pour éliminer les ions aluminium du sang constitué :
- d'un filtre semi-perméable (20), qui présente deux côtés (30, 40),
- d'une circulation sanguine (100, 30, 120) présentant une conduite d'amenée de sang (100) et une conduite de recyclage de sang (120), laquelle circulation de sang est disposée d'un côté (30) du filtre (20),
- d'une conduite de liquide de dialyse (60, 80), qui est disposée de l'autre côté (40) du filtre (20),
- d'une source de liquide de dialyse (50) qui est reliée à la conduite de liquide de dialyse (60), et
- d'une source (150) d'un agent anticoagulant relié à la conduite d'amenée de sang (100), lequel est essentiellement non dialysable,
caractérisé en ce que la source (150) présente de plus un agent complexant les ions aluminium.

10. Dispositif selon la revendication 9, caractérisé en ce que la source (150) présente des quantités prédéterminées de citrate et d'héparine que l'on peut amener à la circulation sanguine (100, 30, 120).

11. Dispositif selon la revendication 9, caractérisé en ce que la source (150) présente des quantités prédéterminées d'héparine essentiellement non dialysable et d'héparine essentiellement dialysable que l'on peut amener au circuit sanguin (100, 30,0 120).

12. Dispositif selon l'une des revendications précédentes, caractérisé en ce que la source de liquide de dialyse (50) présente un liquide de dialyse ayant des concentrations en ions métalliques appropriés à la substitution des ions métalliques complexés, éliminés involontairement de la circulation sanguine (100, 30, 120).

13. Dispositif selon la revendication 12, caractérisé en ce que la source de liquide de dialyse (50) présente des concentrations en ions cuivre, calcium et/ou magnésium, de préférence en ions calcium.

14. Dispositif selon l'une des revendications précédentes caractérisé en ce que, en aval du dialyseur (10), dans la conduite du liquide de dialyse (80), est installée une pompe d'ultrafiltration (230), que l'on peut piloter de façon telle, en ce qu'on peut soutirer, en plus des quantités prédéterminées d'ultrafiltrat, des quantités d'ultrafiltrat à la circulation sanguine (100, 30, 120) correspondant aux quantités de la solution complexant les ions aluminium que l'on amène à la circulation sanguine à partir de l'une des sources (150, 170).

15. Solution complexant les ions aluminium et anticoagulante pour l'utilisation dans un dispositif selon l'une des revendications 9 à 11 ou 12 à 14 en rapport avec une des revendications 9 à 11, caractérisée par une teneur en ions citrate et une teneur en héparine essentiellement non dialysable.

16. Solution selon la revendication 15, caractérisée par une teneur de 40 à 50 g/l, de préférence de 44 g/l en citrate sodique, de 14 à 18 g/l, de préférence de 16 g/l en acide citrique monohydraté et de 30 à 50 mg/l, de préférence de 40 mg/l, en héparine de haut poids moléculaire (PM > 5000).

17. Solution complexant les ions aluminium et anticoagulante pour l'utilisation dans un dispositif selon l'une des revendications 9 à 11 ou 12 à 14 en rapport avec une des revendications 9 à 11, caractérisée par des teneurs en héparine dialysable et en héparine essentiellement non dialysable.

18. Utilisation de la solution selon l'une des revendications 15 à 17 dans un dispositif selon l'une des revendications 9 à 11 ou 12 à 14 en rapport avec une des revedications 9 à 11.
